# EUROPÄISCHE PATENTANMELDUNG

(11) **EP 1 223 429 A1**
(43) Veröffentlichungstag der Anmeldung: **17.07.2002**
(21) Anmeldenummer: 01100773.9
(22) Anmeldetag: 13.01.2001
(51) Int. Cl.: G01N 33/543, C12Q 1/04, G01N 33/569

(54) **Verfahren zum Nachweis von lebenden Mikroorganismen**

(71) Anmelder: Dentognostics GmbH, 47226 Duisburg (DE)
(72) Erfinder: Bauermeister, Claus-Detlev, Dr., 47495 Rheinberg (DE); Miethe, Peter, Dr., c/o Biognosis GmbH, 52428 Jülich (DE)
(74) Vertreter: Meyers, Hans-Wilhelm, Dr.Dipl.-Chem.

(57) **Zusammenfassung**

Verfahren zum Nachweis von lebenden Mikroorganismen, insbesondere einzelligen Organismen, Schimmelpilzen in Proben, wobei die Proben in flüssiger Form mit einem Träger in Kontakt gebracht werden, der für die oder den zu bestimmenden Mikroorganismus spezifische Rezeptoren aufweist, der Mikroorganismus oder die Mikroorganismen an den Rezeptoren festgehalten werden und ein Faktor, der indikativ für lebende Mikroorganismen ist, gemessen wird.

## Beschreibung

Gegenstand der vorliegenden Erfindung ist ein Verfahren zum Nachweis von lebenden Mikroorganismen.

Tests auf das Vorhandensein von lebenden Mikroorganismen sind bei Hygiene-Überwachungen und Untersuchungen an biologischen Materialien, klinischen Proben von Menschen und Tieren, sowie zum Nachweis mikrobieller Kontaminationen, insbesondere bei Hygiene-Überwachungen, Lebensmittel-, Material-, Boden-, Luft-, Wasser- und anderen Umweltuntersuchungen wichtig, da nur lebende Mikroorganismen sich vermehren und biologische Effekte, insbesondere Gesundheitsschäden, hervorrufen können. Gemäß dem Stand der Technik wird durch Aufschluss von Mikroorganismen, die aus kontaminierten oder vermutlich kontaminierten Proben stammen, ATP als Lebensparameter gemessen. Der ATP-Test ist allerdings nicht spezifisch, sondern zeigt nur das Vorhandensein lebender Zellen an. Die auf ATP beruhenden Tests sind insbesondere bei Fleischuntersuchungen aufwendig. Das der Erfindung zu Grunde liegende Problem besteht darin, einen Test zur Verfügung zu stellen, der lebende Mikroorganismen und/oder das Vorhandensein eukaryotischer Zellen anzeigt. Der Test soll dabei gleichzeitig einfach und verläßlich sein.

Das technische Problem wird gelöst durch ein Verfahren gemäß Anspruch 1.

Das erfindungsgemäße Verfahren dient zum Nachweis von lebenden Mikroorganismen, insbesondere einzelligen Organismen, Schimmelpilzen in Proben, wobei die Proben in flüssiger Form mit einem Träger in Kontakt gebracht werden, der für die oder den zu bestimmenden Mikroorganismus spezifische Rezeptoren aufweist, der Mikroorganismus oder die Mikroorganismen an den Rezeptoren festgehalten werden und ein Faktor, der indikativ für lebende Mikroorganismen ist, gemessen wird.

Die bestimmbaren Mikroorganismen sind insbesondere ausgewählt aus der Gruppe umfassend Bakterien, tierische und pflanzliche mindestens einzellige Lebewesen, Pilze, Protozoen und/oder eukaryotische Zellen.

Erfindungsgemäß werden als Probe feste Ausgangsmaterialien verwendet, die in flüssige Phase überführt werden oder Flüssigkeiten umfasst, in denen die Mikroorganismen vermutet werden, insbesondere Körperflüssigkeiten, flüssige Lebensmittel, Spülflüssigkeiten, Abwässer, Aufschwemmungen, Suspensionen, Filtrate, Extrakte, Eluate sowie aus in Trägermaterialien aufgesaugte wiedergewonnene Flüssigkeiten.

Im erfindungsgemäßen Verfahren wird als Träger vorzugsweise ein Material in Form eines Formkörpers, loser Schüttungen in Partikelform, in Form eines Gels oder als Dispersion verwendet, wobei
- das Trägermaterial mindestens einen Rezeptor zur spezifischen Bindung des zu bestimmenden Mikroorganismus trägt und für ein Fluidum durchlässig ist,
- das Trägermaterial einen mittleren Porendurchmesser von 1 bis 200 µm aufweist und einen Porenvolumenanteil von 20 bis 80% aufweist,
- das Trägermaterial keine oder nur eine sehr niedrige unspezifische Sorptionsfähigkeit für die Mikroorganismen hat,
- das Trägermaterial eine Beladung von 10¹² bis 10¹⁸ Rezeptoren pro ml Träger aufweist.

Als Rezeptoren werden insbesondere Antikörper gegen Antigene von Mikroorganismen eingesetzt. Insbesondere werden als Rezeptoren Lectine eingesetzt.

Als Rezeptoren kommen auch Inhibitoren, Substrate, Cofactoren oder Substrat bzw. Cofactor analoge Substanzen von Oberflächenenzymen in Frage.

In einer bevorzugten Ausführungsform des erfindungsgemäßen Verfahrens nach mindestens einem der Ansprüche 1 bis 4, wobei als Rezeptoren Protein A oder Protein G in Kombination mit einem oder mehreren Antikörpern eingesetzt werden.

Erfindungsgemäß sind auch als Rezeptoren, Avidin/Streptavidin in Kombination mit einem oder mehreren biotinylierten Markern einsetzbar.

Gemäß der Erfindung wird als für lebende Mikroorganismen indikativer Faktor ATP mit Luciferase oder NADH im Lysat gemessen.

In einer weiteren Ausführungsform des erfindungsgemäßen Verfahren wird als für lebende Mikroorganismen indikativer Faktor ein Stoff eingesetzt, der in niedriger Konzentration gelöst, nach kurzer Inkubation auf der Säule gebundene lebende Mikroorganismen auf Grund seiner Struktur allein oder in Kombination mit anderen Markern Wechselwirkungen mit DNA oder Zellwandbestandteilen eingeht und eine Unterscheidung lebender von toten Mikroorganismen gestattet und die Detektion auf Grund der Farboder Fluoreszenz-Intensität, die in Folge der Wechselwirkung eintritt, direkt auf der Säule erfolgt.

Erfindungsgemäß kann dann als für lebende Mikroorganismen indikativer Faktor die Aktivität eines Enzyms der auf der Säule gebundenen Mikroorganismen gemessen werden.

In einer alternativen Ausführungsform ist es möglich als für lebende Mikroorganismen indikativer Faktor NADH direkt in den auf der Säule gebundenen Mikroorganismen zu messen.

Das erfindungsgemäße Verfahren erlaubt es, entweder alle Schritte sequentiell ohne zeitliche Unterbrechung zwischen den einzelnen Schritten durchgeführt werden oder nach dem die Mikroorganismen an den Rezeptoren festgehalten worden sind die weitere Abfolge des Verfahrens unterbrochen wird und Bedingungen eingestellt werden unter denen der Zustand der lebenden Mikroorganismen nicht oder nur in reproduzierbarer Weise verändert wird und die nachfolgenden Schritte nach einer Lageroder/und Transportperiode ausgeführt werden.

Der selektive Nachweis von z. B. Streptococcus mutans in einer flüssigen Probe, die ein Gemisch verschiedener Bakterien enthält, wird erreicht durch die spezifische Bindung an sogenannte "capture"-Antikörper, die in miniaturisierten Säulen auf einer Festphase immobilisiert sind. Fließt die bakterienhaltige Probe durch diese Säule, so werden die nachzuweisenden Bakterien vermittels der spezifischen Antikörper selektiv an der Festphase gebunden. Andere, nichtgebunden Bakterien fließen mit der Flüssigkeit aus der Säule heraus bzw. werden durch einen Spülvorgang entfernt. Die nunmehr gebundenen Streptococcus mutans-Bakterien können nachfolgend detektiert werden. Für diese Detektion wird ein hochempfindliches Diolumineszenz-System eingesetzt. Das Meßergebnis steht in Lorrelation zu der Bakterienzahl. Die Sensitivität des liegt bei 100 Mikoorganismen/ml.

## Patentansprüche

1. Verfahren zum Nachweis von lebenden Mikroorganismen und/oder eukaryotischer Zellen, insbesondere einzelligen Organismen, Schimmelpilzen in Proben, wobei die Proben in flüssiger Form mit einem Träger in Kontakt gebracht werden, der für die oder den zu bestimmenden Mikroorganismus spezifische Rezeptoren aufweist, der Mikroorganismus oder die Mikroorganismen an den Rezeptoren festgehalten werden und ein Faktor, der indikativ für lebende Mikroorganismen ist, gemessen wird.

2. Verfahren nach Anspruch 1, wobei die Mikroorganismen ausgewählt sind aus der Gruppe umfassend Bakterien, tierische und pflanzliche mindestens einzellige Lebewesen, Pilze, Protozoen, und/oder eukaryotische Zellen.

3. Verfahren nach Anspruch 1 und/oder 2, wobei die Probe feste Ausgangsmaterialien, die in flüssige Phase überführt werden oder Flüssigkeiten umfasst, in denen die Mikroorganismen vermutet werden, insbesondere Körperflüssigkeiten, flüssige Lebensmittel, Spülflüssigkeiten, Abwässer, Aufschwemmungen, Suspensionen, Filtrate, Extrakte, Eluate sowie aus in Trägermaterialien aufgesaugte wiedergewonnene Flüssigkeiten.

4. Verfahren nach mindestens einem der Ansprüche 1 bis 3, wobei als Träger ein Material in Form eines Formkörpers, loser Schüttungen in Partikelform, in Form eines Gels oder als Dispersion, wobei
• das Trägermaterial mindestens einen Rezeptor zur spezifischen Bindung des zu bestimmenden Mikroorganismus trägt und für ein Fluidum durchlässig ist,
• das Trägermaterial einen mittleren Porendurchmesser von 1 bis 200 µm aufweist und einen Porenvolumenanteil von 20 bis 80% aufweist,
• das Trägermaterial keine oder nur eine sehr niedrige unspezifische Sorptionsfähigkeit für die Mikroorganismen hat,
• das Trägermaterial eine Beladung von 10¹² bis 10¹⁸ Rezeptoren pro ml Träger aufweist.

5. Verfahren nach mindestens einem der Ansprüche 1 bis 4, wobei als Rezeptoren Antikörper gegen Oberflächenantigene von Mikroorganismen eingesetzt werden.

6. Verfahren nach mindestens einem der Ansprüche 1 bis 4, wobei als Rezeptoren Lectine eingesetzt werden.

7. Verfahren nach mindestens einem der Ansprüche 1 bis 4, wobei als Rezeptoren Inhibitoren, Substrate, Cofactoren oder Substrat bzw. Cofactor analoge Substanzen von Oberflächenenzymen eingesetzt werden.

8. Verfahren nach mindestens einem der Ansprüche 1 bis 4, wobei als Rezeptoren Protein A oder Protein G in Kombination mit einem oder mehreren Antikörpern eingesetzt werden

9. Verfahren nach mindestens einem der Ansprüche 1 bis 4, wobei als Rezeptoren, Avidin/Streptavidin in Kombination mit einem oder mehreren biotinylierten Markern eingesetzt werden.

10. Verfahren nach mindestens einem der Ansprüche 1 bis 10, wobei als für lebende Mikroorganismen indikativer Faktor ATP mit Luciferase oder NADH im Lysat gemessen wird.

11. Verfahren nach mindestens einem der Ansprüche 1 bis 10 wobei als für lebende Mikroorganismen indikativer Faktor ein Stoff, der in niedriger Konzentration gelöst nach kurzer Inkubation auf der Säule gebundene lebende Mikroorganismen auf Grund seiner Struktur allein oder in Kombination mit anderen Markern Wechselwirkungen mit DNA oder Zellwandbestandteilen aufweist und eine Unterscheidung lebender von toten Mikroorganismen gestattet, verwendet wird und die Detektion auf Grund seiner Farb- oder Fluoreszenz-Intensität direkt auf der Säule erfolgt.

12. Verfahren nach mindestens einem der Ansprüche 1 bis 10 wobei als für lebende Mikroorganismen indikativer Faktor die Aktivität eines Enzyms der auf der Säule gebundenen Mikroorganismen gemessen wird.

13. Verfahren nach mindestens einem der Ansprüche 1 bis 10 wobei als für lebende Mikroorganismen indikativer Faktor NADH direkt in den auf der Säule gebundenen Mikroorganismen gemessen wird.

14. Verfahren nach einem der Ansprüche 1 bis 8 wobei
a) entweder alle Schritte sequentiell ohne zeitliche Unterbrechung zwischen den einzelnen Schritten durchgeführt werden oder
b) nach dem die Mikroorganismen an den Rezeptoren festgehalten worden sind die weitere Abfolge des Verfahrens unterbrochen wird und Bedingungen eingestellt werden unter denen der Zustand der lebenden Mikroorganismen nicht oder nur in reproduzierbarer Weise verändert wird und die nachfolgenden Schritte nach einer Lageroder/und Transportperiode ausgeführt werden.
